# EUROPEAN PATENT APPLICATION

(11) **EP 2 995 608 A1**
(43) Date of publication of application: **16.03.2016**
(21) Application number: 15186556.5
(22) Date of filing: 03.12.2010
(51) Int. Cl.: C07C 211/61

(54) **DEUTERATED COMPOUNDS FOR LUMINESCENT APPLICATIONS**

(30) Priority: 21.12.2009 US 643257
(62) Divisional of application: 10842446.6
(71) Applicant: E. I. du Pont de Nemours and Company, Wilmington, DE 19805 (US)
(72) Inventor: LECLOUX, Daniel, David, Cary, NC 27513 (US); MENG, Hong, Wilmington, DE 19805 (US)
(74) Representative: Towler, Philip Dean

(57) **Abstract**

This invention relates to deuterated compounds that are useful in electroluminescent applications. It also relates to electronic devices in which the active layer includes such a deuterated compound.

## Description

### BACKGROUND

### Field of the Disclosure

This invention relates to electroactive compounds which are at least partially deuterated. It also relates to electronic devices in which the active layers include such a compound.

### Description of the Related Art

Organic electronic devices that emit light, such as light-emitting diodes that make up displays, are present in many different kinds of electronic equipment. In all such devices, an organic active layer is sandwiched between two electrical contact layers. At least one of the electrical contact layers is light-transmitting so that light can pass through the electrical contact layer. The organic active layer emits light through the light-transmitting electrical contact layer upon application of electricity across the electrical contact layers.

It is well known to use organic electroluminescent compounds as the active component in light-emitting diodes. Simple organic molecules such as anthracene, thiadiazole derivatives, and coumarin derivatives are known to show electroluminescence. Semiconductive conjugated polymers have also been used as electroluminescent components, as has been disclosed in, for example, U.S. Patent 5,247,190, U.S. Patent 5,408,109, and Published European Patent Application 443 861.

However, there is a continuing need for electroluminescent compounds.

### SUMMARY

There is provided a compound having Formula I

Q-(NAr₂)ₐ Formula I

where:
Q is an aromatic core selected from the group consisting of benz[a]anthracene, dibenz[a,h]anthracene, fluoranthene, fluorene, naphthalene, perylene, phenanthrene, pyrene, spirofluorene, tetracene, and substituted derivatives thereof;
Ar is aryl; and
a is 1 or 2;
wherein the compound has at least one D.

There is also provided an electronic device comprising an active layer comprising the above compound.

### BRIEF DESCRIPTION OF THE DRAWINGS

Embodiments are illustrated in the accompanying figures to improve understanding of concepts as presented herein.
FIG. 1 includes an illustration of one example of an organic electronic device.
FIG. 2 includes another illustration of an organic electronic device.

Skilled artisans appreciate that objects in the figures are illustrated for simplicity and clarity and have not necessarily been drawn to scale. For example, the dimensions of some of the objects in the figures may be exaggerated relative to other objects to help to improve understanding of embodiments.

### DETAILED DESCRIPTION

Many aspects and embodiments are disclosed herein and are exemplary and not limiting. After reading this specification, skilled artisans appreciate that other aspects and embodiments are possible without departing from the scope of the invention.

Other features and benefits of any one or more of the embodiments will be apparent from the following detailed description, and from the claims. The detailed description first addresses Definitions and Clarification of Terms followed by the Electroactive Compound, and the Electronic Device.

### 1. Definitions and Clarification of Terms

Before addressing details of embodiments described below, some terms are defined or clarified.

As used herein, the term "aliphatic ring" is intended to mean a cyclic group that does not have delocalized pi electrons. In some embodiments, the aliphatic ring has no unsaturation. In some embodiments, the ring has one double or triple bond.

The term "alkoxy" refers to the group RO-, where R is an alkyl.

The term "alkyl" is intended to mean a group derived from an aliphatic hydrocarbon having one point of attachment, and includes a linear, a branched, or a cyclic group. The term is intended to include heteroalkyls and deuterated alkyls. The term is intended to include substituted and unsubstituted groups. The term "hydrocarbon alkyl" refers to an alkyl group having no heteroatoms. The term "deuterated alkyl" is a hydrocarbon alkyl having at least one available H replaced by D. In some embodiments, an alkyl group has from 1-20 carbon atoms.

The term "aryl" is intended to mean a group derived from an aromatic hydrocarbon having one point of attachment. The term "aromatic compound" is intended to mean an organic compound comprising at least one unsaturated cyclic group having delocalized pi electrons. The term is intended to include heteroaryls and deuterated aryls. The term "hydrocarbon aryl" is intended to mean aromatic compounds having no heteroatoms in the ring. The term aryl includes groups which have a single ring and those which have multiple rings which can be joined by a single bond or fused together. The term "deuterated aryl" refers to an aryl group having at least one of the available H atoms which is bonded directly to the aryl replaced by D. The term "arylene" is intended to mean a group derived from an aromatic hydrocarbon having two points of attachment. Any suitable ring position of the aryl moiety may be covalently linked to the defined chemical structure. In some embodiments, a hydrocarbon aryl group has from 3-60 carbon atoms; in some embodiments, 6 to 30 carbon atoms. Heteroaryl groups may have from 3-50 carbon atoms; in some embodiments, 3-30 carbon atoms.

The term "branched alkyl" refers to an alkyl group having at least one secondary or tertiary carbon. The term "secondary alkyl" refers to a branched alkyl group having a secondary carbon atom. The term "tertiary alkyl" refers to a branched alkyl group having a tertiary carbon atom. In some embodiments, the branched alkyl group is attached via a secondary or tertiary carbon.

The term "charge transport," when referring to a layer, material, member, or structure is intended to mean such layer, material, member, or structure facilitates migration of such charge through the thickness of such layer, material, member, or structure with relative efficiency and small loss of charge. Hole transport materials facilitate positive charge; electron transport material facilitate negative charge. Although light-emitting materials may also have some charge transport properties, the terms "charge, hole, or electron transport layer, material, member, or structure" are not intended to include a layer, material, member, or structure whose primary function is light emission.

The term "compound" is intended to mean an electrically uncharged substance made up of molecules that further consist of atoms, wherein the atoms cannot be separated by physical means. The phrase "adjacent to," when used to refer to layers in a device, does not necessarily mean that one layer is immediately next to another layer. On the other hand, the phrase "adjacent R groups," is used to refer to R groups that are next to each other in a chemical formula (i.e., R groups that are on atoms joined by a bond). The term "electroactive" refers to any material that exhibits electroluminescence and/or photosensitivity.

The term "deuterated" is intended to mean that at least one available H has been replaced by D. A compound or group that is X% deuterated, has X% of the available H replaced by D. A compound or group which is deuterated is one in which deuterium is present in at least 100 times the natural abundance level.

The term "electroactive" as it refers to a layer or a material, is intended to indicate a layer or material which electronically facilitates the operation of the device. Examples of active materials include, but are not limited to, materials which conduct, inject, transport, or block a charge, where the charge can be either an electron or a hole, or materials which emit radiation or exhibit a change in concentration of electron-hole pairs when receiving radiation. Examples of inactive materials include, but are not limited to, planarization materials, insulating materials, and environmental barrier materials.

The prefix "hetero" indicates that one or more carbon atoms have been replaced with a different atom. In some embodiments, the different atom is N, O, or S.

The term "layer" is used interchangeably with the term "film" and refers to a coating covering a desired area. The term is not limited by size. The area can be as large as an entire device or as small as a specific functional area such as the actual visual display, or as small as a single sub-pixel. Layers and films can be formed by any conventional deposition technique, including vapor deposition, liquid deposition (continuous and discontinuous techniques), and thermal transfer. Continuous deposition techniques, include but are not limited to, spin coating, gravure coating, curtain coating, dip coating, slot-die coating, spray coating, and continuous nozzle coating. Discontinuous deposition techniques include, but are not limited to, ink jet printing, gravure printing, and screen printing.

The term "organic electronic device" or sometimes just "electronic device" is intended to mean a device including one or more organic semiconductor layers or materials.

The term "oxyalkyl" is intended to mean a heteroalkyl group having one or more carbons replaced with oxygens. The term includes groups which are linked via an oxygen.

The term "silyl" refers to the group R₃Si-, where R is H, D, C1-20 alkyl, fluoroalkyl, or aryl. In some embodiments, one or more carbons in an R alkyl group are replaced with Si. In some embodiments, the silyl groups are (hexyl)₂Si(Me)CH₂CH₂Si(Me)₂- and [CF₃(CF₂)₆CH₂CH₂]₂SiMe-.

The term "siloxane" refers to the group (RO)₃Si-, where R is H, D , C1-20 alkyl, or fluoroalkyl.

All groups can be substituted or unsubstituted unless otherwise indicated. In some embodiments, the substituents are selected from the group consisting of D, halide, alkyl, alkoxy, aryl, and cyano. An optionally substituted group, such as, but not limited to, alkyl or aryl, may be substituted with one or more substituents which may be the same or different. Other suitable substituents include nitro, cyano, -N(R')(R"), hydroxy, carboxy, alkenyl, alkynyl, aryloxy, alkoxycarbonyl, perfluoroalkyl, perfluoroalkoxy, arylalkyl, silyl, siloxane, thioalkoxy, -S(O)₂-N(R')(R"),-C(=O)-N(R')(R"), (R')(R")N-alkyl, (R')(R")N-alkoxyalkyl, (R')(R")N-alkylaryloxyalkyl, -S(O)ₛ-aryl (where s=0-2) or -S(O)ₛ-heteroaryl (where s=0-2). Each R' and R" is independently an optionally substituted alkyl, cycloalkyl, or aryl group. R' and R", together with the nitrogen atom to which they are bound, can form a ring system in certain embodiments. Substituents may also be crosslinking groups.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The IUPAC numbering system is used throughout, where the groups from the Periodic Table are numbered from left to right as 1-18 (CRC Handbook of Chemistry and Physics, 81st Edition, 2000).

### 2. Electroactive Compound

The compound described herein is a deuterated aromatic compound having at least one diarylamino substituent. In some embodiments, the compounds are electroluminescent and are capable of red, green or blue emission.

In some embodiments, the compound is at least 10% deuterated; in some embodiments, at least 20% deuterated; in some embodiments, at least 30% deuterated; in some embodiments, at least 40% deuterated; in some embodiments, at least 50% deuterated; in some embodiments, at least 60% deuterated; in some embodiments, at least 70% deuterated; in some embodiments, at least 80% deuterated; in some embodiments, at least 90% deuterated. In some embodiments, the compound is 100% deuterated.

The deuteration can be present on one or more areas selected from a substituent group on an aryl ring, aryl rings Ar¹ through Ar⁴, and the core Q group.

In some embodiments of Formula I, the deuteration is on a substituent group on an aryl ring. The aryl group having a deuterated substituent group can be can be any one or more of: the core Q group; an aryl on the nitrogen; or a substituent aryl group. In some embodiments, the deuterated substituent group on an aryl ring is selected from alkyl, aryl, alkoxy, and aryloxy. In some embodiments, the substituent groups are at least 10% deuterated; in some embodiments, at least 20% deuterated; in some embodiments, at least 30% deuterated; in some embodiments, at least 40% deuterated; in some embodiments, at least 50% deuterated; in some embodiments, at least 60% deuterated; in some embodiments, at least 70% deuterated; in some embodiments, at least 80% deuterated; in some embodiments, at least 90% deuterated; in some embodiments, 100% deuterated.

In some embodiments of Formula I, the deuteration is on any one or more of the aryl groups Ar¹ through Ar⁴. In this case, at least one of Ar¹ through Ar⁴ is a deuterated aryl group. In some embodiments, Ar¹ through Ar⁴ are at least 10% deuterated. By this it is meant that at least 10% of all the available H bonded to aryl C in Ar¹ through Ar⁴ are replaced with D. In some embodiments, each aryl ring will have some D. In some embodiments, some, and not all of the aryl rings have D. In some embodiments, Ar¹ through Ar⁴ are at least 20% deuterated; in some embodiments, at least 30% deuterated; in some embodiments, at least 40% deuterated; in some embodiments, at least 50% deuterated; in some embodiments, at least 60% deuterated; in some embodiments, at least 70% deuterated; in some embodiments, at least 80% deuterated; in some embodiments, at least 90% deuterated; in some embodiments, 100% deuterated.

In some embodiments of Formula I, the deuteration is present on the core Q group. In some embodiments, the Q group is at least 20% deuterated; in some embodiments, at least 30% deuterated; in some embodiments, at least 40% deuterated; in some embodiments, at least 50% deuterated; in some embodiments, at least 60% deuterated; in some embodiments, at least 70% deuterated; in some embodiments, at least 80% deuterated; in some embodiments, at least 90% deuterated; in some embodiments, 100% deuterated.

In some embodiments of Formula I, Q is a benz[a]anthracene. In some embodiments, the compound has formula II 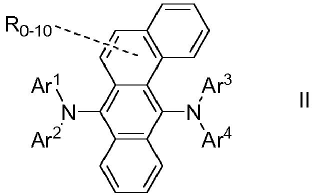 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁴ are the same or different and are selected from the group consisting of aryl groups;
wherein the compound has at least one D.

The dashed line in the formula is intended to indicate that the R group, when present, can be at any site on the benz[a]anthracene core.

In some embodiments of formula II, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula II, at least one of Ar¹ through Ar⁴ has formula (a): 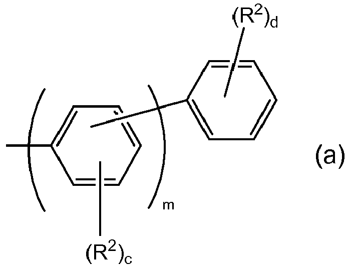 where:
R² is the same or different at each occurrence and is selected from the group consisting of D, alkyl, alkoxy, aryl, silyl, and siloxane, or adjacent R² groups can be joined to form an aromatic ring;
c is the same or different at each occurrence and is an integer from 0-4;
d is the same or different at each occurrence and is an integer from 0-5; and
m is the same or different at each occurrence and is an integer from 0 to 6.

In some embodiments of formula II, at least one of Ar¹ through Ar⁴ has Formula (b): 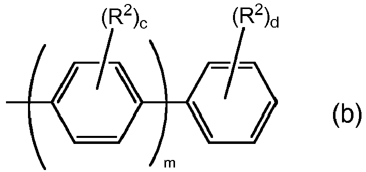 where:
R² is the same or different at each occurrence and is selected from the group consisting of D, alkyl, alkoxy, and aryl, or adjacent R² groups can be joined to form an aromatic ring;
c is the same or different at each occurrence and is an integer from 0-4;
d is the same or different at each occurrence and is an integer from 0-5; and
m is the same or different at each occurrence and is an integer from 0 to 6.

In some embodiments of formula II, Ar1 through Ar4 are selected from the group consisting of phenyl, biphenyl, terphenyl, naphthyl, phenylnapthyl, naphthylphenyl, binaphthyl, and deuterated analogs thereof.

In some embodiments of formula II, Ar¹ through Ar⁴ are perdeuterated.

In some embodiments of formula II, Ar¹ through Ar⁴ are perdeuterated, except for one alkyl group on a terminal aryl.

In some embodiments of formula II, the compound is not symmetrical with respect to the Ar groups and Ar¹ is not the same as either Ar³ or Ar⁴.

In some embodiments of Formula I, Q is a dibenz[a,h]anthracene. In some embodiments, the compound has formula III 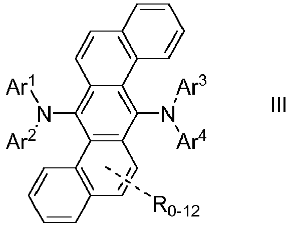 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁴ are the same or different and are selected from the group consisting of aryl groups;
wherein the compound has at least one D.

The dashed line in the formula is intended to indicate that the R group, when present, can be at any site on the dibenz[a,h]anthracene core.

In some embodiments of formula III, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula III, at least one of Ar¹ through Ar⁴ has formula (a), as described above. In some embodiments of formula III, at least one of Ar¹ through Ar⁴ has Formula (b), as described above.

In some embodiments of formula III, Ar1 through Ar4 are selected from the group consisting of phenyl, biphenyl, terphenyl, naphthyl, phenylnapthyl, naphthylphenyl, binaphthyl, and deuterated analogs thereof.

In some embodiments of formula III, Ar¹ through Ar⁴ are perdeuterated.

In some embodiments of formula III, Ar¹ through Ar⁴ are perdeuterated, except for one alkyl group on a terminal aryl.

In some embodiments of Formula I, Q is a fluoranthene. In some embodiments, the compound has formula IV 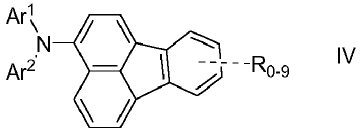 wherein:
R is the same or different at each occurrence and is selected from the group consisting of D, alkyl, alkoxy and aryl, where adjacent R groups may be joined together to form a 5- or 6-membered aliphatic ring;
Ar¹ and Ar² are the same or different and are selected from the group consisting of aryl groups;
wherein the compound has at least one D.

The dashed line in the formula is intended to indicate that the R group, when present, can be at any site on the fluoranthene core.

In some embodiments of formula IV, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula IV, at least one of Ar¹ and Ar² has formula (a), as described above. In some embodiments of formula III, at least one of Ar¹ and Ar² has Formula (b), as described above.

In some embodiments of formula IV, Ar¹ and Ar² are selected from the group consisting of phenyl, biphenyl, terphenyl, naphthyl, phenylnapthyl, naphthylphenyl, binaphthyl, and deuterated analogs thereof.

In some embodiments of formula IV, Ar¹ and Ar² are perdeuterated.

In some embodiments of formula IV, Ar¹ and Ar² are perdeuterated, except for one alkyl group on a terminal aryl.

In some embodiments of formula IV, Ar¹ is not the same as Ar².

In some embodiments of Formula I, Q is a fluorene. In some embodiments, the compound has formula V 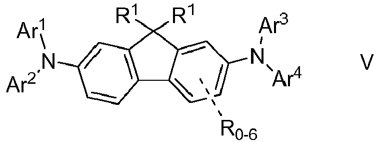 wherein:
R is the same or different at each occurrence and is selected from the group consisting of D, alkyl, alkoxy and aryl, where adjacent R groups may be joined together to form a 5- or 6-membered aliphatic ring;
R¹ is the same or different at each occurrence and is selected from alkyl groups;
Ar¹ through Ar⁴ are the same or different and are selected from the group consisting of aryl groups;
wherein the compound has at least one D.

The dashed line in the formula is intended to indicate that the R group, when present, can be at any site on the fluorene core.

In some embodiments of formula V, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula V, R¹ is a C1-10 alkyl group. In some embodiments of formula V, the R¹ groups are the same.

In some embodiments of formula V, at least one of Ar¹ through Ar⁴ has formula (a), as described above. In some embodiments of formula III, at least one of Ar¹ through Ar⁴ has Formula (b), as described above.

In some embodiments of formula V, Ar¹ through Ar⁴ are selected from the group consisting of phenyl, biphenyl, terphenyl, naphthyl, phenylnapthyl, naphthylphenyl, binaphthyl, and deuterated analogs thereof.

In some embodiments of formula V, Ar¹ through Ar⁴ are perdeuterated.

In some embodiments of formula V, Ar¹ through Ar⁴ are perdeuterated, except for one alkyl group on a terminal aryl.

In some embodiments of formula V, the compound is not symmetrical with respect to the Ar groups and Ar¹ is not the same as either Ar³ or Ar⁴.

In some embodiments of Formula I, Q is a naphthalene. In some embodiments, the compound has a formula selected from VI-a, VI-b, and VI-c. 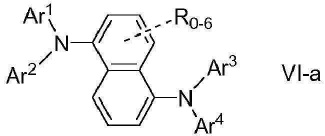 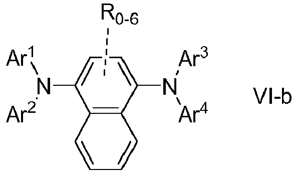 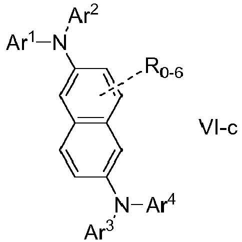 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁴ are the same or different and are selected from the group consisting of aryl groups;
wherein the compound has at least one D.

The dashed line in the formula is intended to indicate that the R group, when present, can be at any site on the naphthalene core. Formulae VI-a, VI-b and VI-c will be referred to collectively as formula VI.

In some embodiments of formula VI, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula VI, at least one of Ar¹ through Ar⁴ has formula (a), as described above. In some embodiments of formula III, at least one of Ar¹ through Ar⁴ has Formula (b), as described above.

In some embodiments of formula VI, Ar1 through Ar4 are selected from the group consisting of phenyl, biphenyl, terphenyl, naphthyl, phenylnapthyl, naphthylphenyl, binaphthyl, and deuterated analogs thereof.

In some embodiments of formula VI, Ar¹ through Ar⁴ are perdeuterated.

In some embodiments of formula VI, Ar¹ through Ar⁴ are perdeuterated, except for one alkyl group on a terminal aryl.

In some embodiments of formula VI, the compound is not symmetrical with respect to the Ar groups and Ar¹ is not the same as either Ar³ or Ar⁴.

In some embodiments of Formula I, Q is a perylene. In some embodiments, the compound has formula VII-a or VII-b. 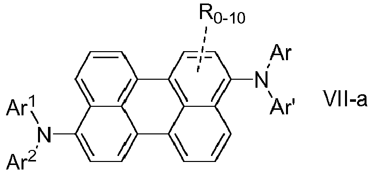 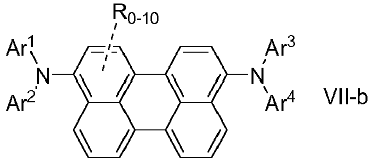 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁴ are the same or different and are selected from the group consisting of aryl groups;
wherein the compound has at least one D.

The dashed line in the formula is intended to indicate that the R group, when present, can be at any site on the perylene core. Formulae VII-a and VII-b will be referred to collectively as formula VII.

In some embodiments of formula VII, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula VII, at least one of Ar¹ through Ar⁴ has formula (a), as described above. In some embodiments of formula III, at least one of Ar¹ through Ar⁴ has Formula (b), as described above.

In some embodiments of formula VII, Ar1 through Ar4 are selected from the group consisting of phenyl, biphenyl, terphenyl, naphthyl, phenylnapthyl, naphthylphenyl, binaphthyl, and deuterated analogs thereof.

In some embodiments of formula VII, Ar¹ through Ar⁴ are perdeuterated.

In some embodiments of formula VII, Ar¹ through Ar⁴ are perdeuterated, except for one alkyl group on a terminal aryl.

In some embodiments of formula VII, the compound is not symmetrical with respect to the Ar groups and Ar¹ is not the same as either Ar³ or Ar⁴.

In some embodiments of Formula I, Q is a phenanthrene. In some embodiments, the compound has formula VIII-a or VIII-b. 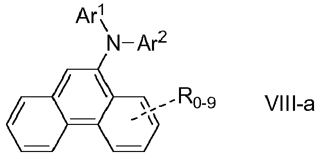 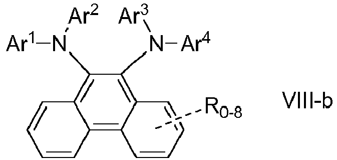 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁴ are the same or different and are selected from the group consisting of aryl groups;
wherein the compound has at least one D.

The dashed line in the formula is intended to indicate that the R group, when present, can be at any site on the phenanthrene core. Formulae VIII-a and VIII-b will be referred to collectively as formula VIII.

In some embodiments of formula VIII, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula VIII, at least one of Ar¹ through Ar⁴ has formula (a), as described above. In some embodiments of formula III, at least one of Ar¹ through Ar⁴ has Formula (b), as described above.

In some embodiments of formula VIII, Ar¹ through Ar⁴ are selected from the group consisting of phenyl, biphenyl, terphenyl, naphthyl, phenylnapthyl, naphthylphenyl, binaphthyl, and deuterated analogs thereof.

In some embodiments of formula VIII, Ar¹ through Ar⁴ are perdeuterated.

In some embodiments of formula VIII, Ar¹ through Ar⁴ are perdeuterated, except for one alkyl group on a terminal aryl.

In some embodiments of formula VIII-a, Ar¹ is not the same as Ar².

In some embodiments of formula VIII-b, the compound is not symmetrical with respect to the Ar groups and Ar¹ is not the same as either Ar³ or Ar⁴.

In some embodiments of Formula I, Q is a pyrene. In some embodiments, the compound has formula IX-a, IX-b, or IX-c. 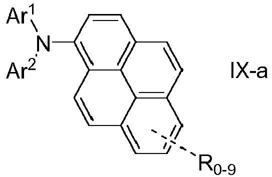 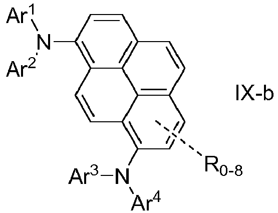 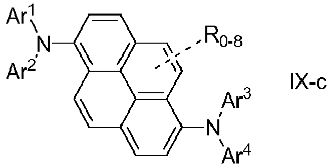 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁴ are the same or different and are selected from the group consisting of aryl groups;
wherein the compound has at least one D.

The dashed line in the formula is intended to indicate that the R group, when present, can be at any site on the pyrene core. Formulae IX-a through IX-c will be referred to collectively as formula IX.

In some embodiments of formula IX, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula IX, at least one of Ar¹ through Ar⁴ has formula (a), as described above. In some embodiments of formula III, at least one of Ar¹ through Ar⁴ has Formula (b), as described above.

In some embodiments of formula IX, Ar¹ through Ar⁴ are selected from the group consisting of phenyl, biphenyl, terphenyl, naphthyl, phenylnapthyl, naphthylphenyl, binaphthyl, and deuterated analogs thereof.

In some embodiments of formula IX, Ar¹ through Ar⁴ are perdeuterated.

In some embodiments of formula IX, Ar¹ through Ar⁴ are perdeuterated, except for one alkyl group on a terminal aryl.

In some embodiments of formula IX-a, Ar¹ is not the same as Ar².

In some embodiments of formula IX-b and IX-c, the compound is not symmetrical with respect to the Ar groups and Ar¹ is not the same as either Ar³ or Ar⁴.

In some embodiments of Formula I, Q is a spirofluorene. In some embodiments, the compound has formula X 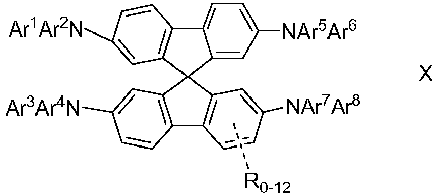 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁸ are the same or different and are selected from the group consisting of aryl groups;
wherein the compound has at least one D.

The dashed line in the formula is intended to indicate that the R group, when present, can be at any site on the spirofluorene core.

In some embodiments of formula X, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula X, at least one of Ar¹ through Ar⁸ has formula (a), as described above. In some embodiments of formula III, at least one of Ar¹ through Ar⁸ has Formula (b), as described above.

In some embodiments of formula X, Ar¹ through Ar⁸ are selected from the group consisting of phenyl, biphenyl, terphenyl, naphthyl, phenylnapthyl, naphthylphenyl, binaphthyl, and deuterated analogs thereof.

In some embodiments of formula X, Ar¹ through Ar⁸ are perdeuterated.

In some embodiments of formula X, Ar¹ through Ar⁸ are perdeuterated, except for one alkyl group on a terminal aryl.

In some embodiments of formula X, the compound is not symmetrical with respect to the Ar groups. In some embodiments, Ar¹ and Ar², collectively are not the same as either Ar⁵ and Ar⁶, collectively, or Ar⁷ and Ar⁸, collectively. In some embodiments, Ar¹ is not the same as any of Ar⁵ through Ar⁸.

In some embodiments of Formula I, Q is a tetracene. In some embodiments, the compound has formula XI 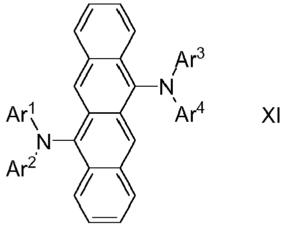 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁴ are the same or different and are selected from the group consisting of aryl groups;
wherein the compound has at least one D.

The dashed line in the formula is intended to indicate that the R group, when present, can be at any site on the tetracene core.

In some embodiments of formula XI, at least one R is a hydrocarbon alkyl. In some embodiments, R is a deuterated alkyl. In some embodiments, R is selected from a branched hydrocarbon alkyl, a cyclic hydrocarbon alkyl, and deuterated analogs thereof.

In some embodiments of formula XI, at least one of Ar¹ through Ar⁴ has formula (a), as described above. In some embodiments of formula III, at least one of Ar¹ through Ar⁴ has Formula (b), as described above.

In some embodiments of formula XI, Ar¹ through Ar⁴ are selected from the group consisting of phenyl, biphenyl, terphenyl, naphthyl, phenylnapthyl, naphthylphenyl, binaphthyl, and deuterated analogs thereof.

In some embodiments of formula XI, Ar¹ through Ar⁴ are perdeuterated.

In some embodiments of formula XI, Ar¹ through Ar⁴ are perdeuterated, except for one alkyl group on a terminal aryl.

In some embodiments of formula XI, the compound is not symmetrical with respect to the Ar groups and Ar¹ is not the same as either Ar³ or Ar⁴.

Examples of compounds having Formula I include, but are not limited to, those shown below.

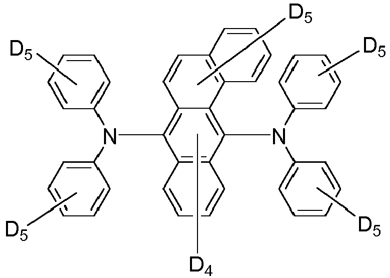

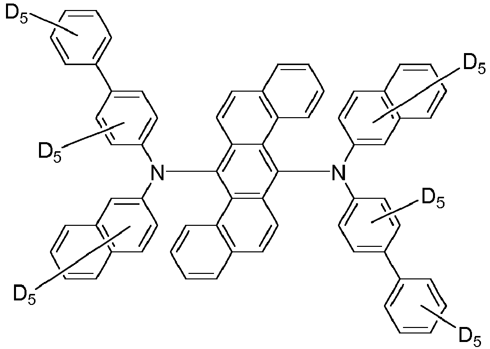

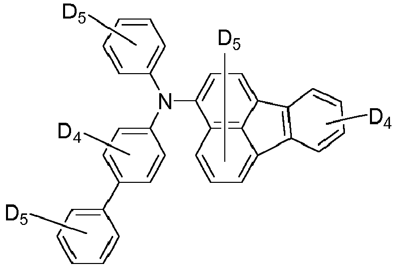

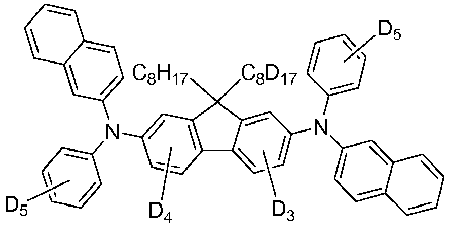

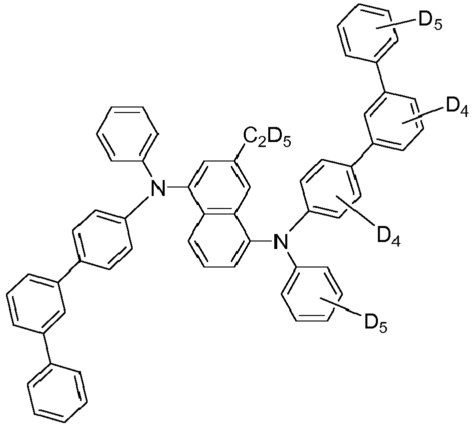

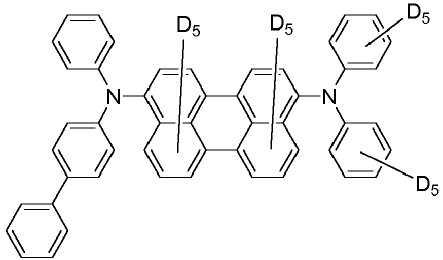

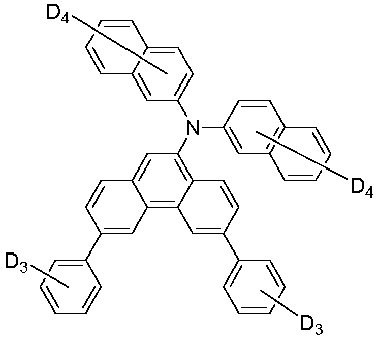

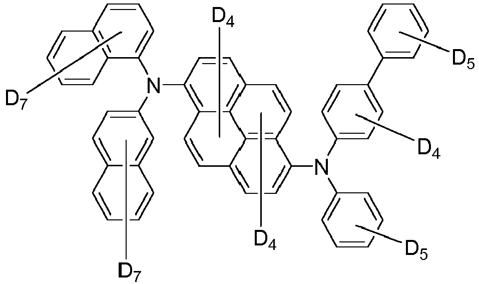

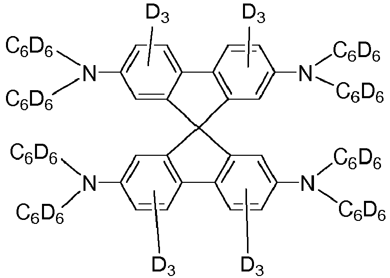

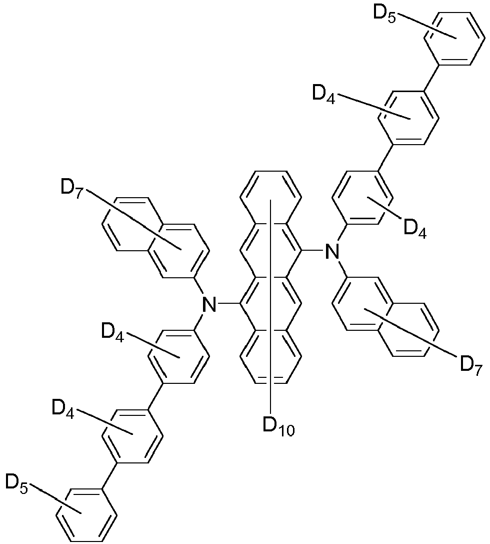

The non-deuterated analogs of the new compounds can be prepared by known coupling and substitution reactions. The new deuterated compound can then be prepared in a similar manner using deuterated precursor materials or, more generally, by treating the non-deuterated compound with deuterated solvent, such as d6-benzene, in the presence of a Lewis acid H/D exchange catalyst, such as aluminum trichloride or ethyl aluminum chloride, or acids such as CF₃COOD, DCI, etc. The level of deuteration can be determined by NMR analysis and by mass spectrometry, such as Atmospheric Solids Analysis Probe Mass Spectrometry **(*ASAP-MS*).** The starting materials of the perdeuterated or partially deuterated aromatic compounds or alkyl compounds can be purchased from commercial sources or can be obtained using known methods. Some examples of such methods can be found in a) "Efficient H/D Exchange Reactions of Alkyl-Substituted Benzene Derivatives by Means of the Pd/C-H2-D2O System" Hiroyoshi Esaki, Fumiyo Aoki, Miho Umemura, Masatsugu Kato, Tomohiro Maegawa, Yasunari Monguchi, and Hironao Sajiki Chem. Eur. J. 2007, 13, 4052 - 4063. b) "Aromatic H/D Exchange Reaction Catalyzed by Groups 5 and 6 Metal Chlorides" GUO, Qiao-Xia, SHEN, Bao-Jian; GUO, Hai-Qing TAKAHASHI, Tamotsu Chinese Journal of Chemistry, 2005, 23, 341-344; c) "A novel deuterium effect on dual charge-transfer and ligand-field emission of the cis-dichlorobis(2,2'-bipyridine)iridium(III) ion" Richard J. Watts, Shlomo Efrima, and Horia Metiu J. Am. Chem. Soc., 1979, 101 (10), 2742-2743; d) "Efficient H-D Exchange of Aromatic Compounds in Near-Critical D20 Catalysed by a Polymer-Supported Sulphonic Acid" Carmen Boix and Martyn Poliakoff Tetrahedron Letters 40 (1999) 4433-4436; e) US3849458; f) "Efficient C-H/C-D Exchange Reaction on the Alkyl Side Chain of Aromatic Compounds Using Heterogeneous Pd/C in D2O" Hironao Sajiki, Fumiyo Aoki, Hiroyoshi Esaki, Tomohiro Maegawa, and Kosaku Hirota Org. Lett., 2004, 6 (9), 1485-1487.

The compounds described herein can be formed into films using liquid deposition techniques. Surprisingly and unexpectedly, these compounds have greatly improved properties when compared to analogous non-deuterated compounds. Electronic devices including an active layer with the compounds described herein, have greatly improved lifetimes. In addition, the lifetime increases are achieved in combination with high quantum efficiency and good color saturation. Furthermore, the deuterated compounds described herein have greater air tolerance than the non-deuterated analogs. This can result in greater processing tolerance both for the preparation and purification of the materials and in the formation of electronic devices using the materials.

The new deuterated compounds described herein have utility as hole transport materials, as electroluminescent materials, and as hosts for electroluminescent materials.

### 3. Electronic Device

Organic electronic devices that may benefit from having one or more layers comprising the deuterated materials described herein include, but are not limited to, (1) devices that convert electrical energy into radiation (e.g., a light-emitting diode, light emitting diode display, or diode laser), (2) devices that detect signals through electronics processes (e.g., photodetectors, photoconductive cells, photoresistors, photoswitches, phototransistors, phototubes, IR detectors), (3) devices that convert radiation into electrical energy, (e.g., a photovoltaic device or solar cell), and (4) devices that include one or more electronic components that include one or more organic semi-conductor layers (e.g., a transistor or diode).

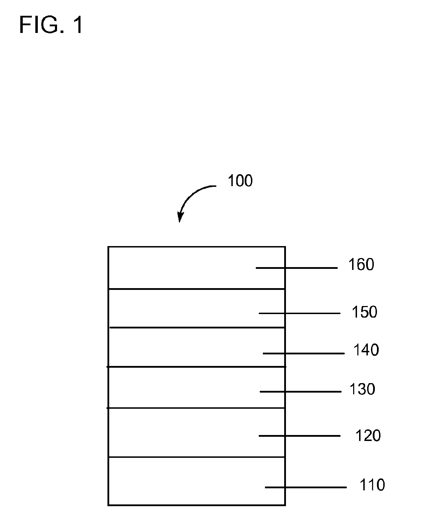

One illustration of an organic electronic device structure is shown in FIG. 1. The device 100 has a first electrical contact layer, an anode layer 110 and a second electrical contact layer, a cathode layer 160, and an electroactive layer 140 between them. Adjacent to the anode is a hole injection layer 120. Adjacent to the hole injection layer is a hole transport layer 130, comprising hole transport material. Adjacent to the cathode may be an electron transport layer 150, comprising an electron transport material. As an option, devices may use one or more additional hole injection or hole transport layers (not shown) next to the anode 110 and/or one or more additional electron injection or electron transport layers (not shown) next to the cathode 160.

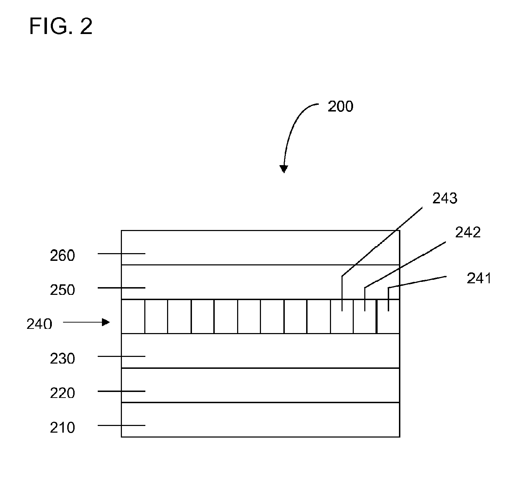

In some embodiments, in order to achieve full color, the light-emitting layer is pixellated, with subpixel units for each of the different colors. An illustration of a pixellated device is shown in FIG. 2. The device 200 has anode 210, hole injection layer 220, hole transport layer 230, electroluminescent layer 240, electron transport layer 250, and cathode 260. The electroluminescent layer is divided into subpixels 241, 242, 243, which are repeated across the layer. In some embodiments, the subpixels represent red, blue and green color emission. Although three different subpixel units are depicted in FIG. 2, two or more than three subpixel units may be used.

The different layers will be discussed further herein with reference to FIG. 1. However, the discussion applies to FIG. 2 and other configurations as well.

Layers 120 through 150 are individually and collectively referred to as the active layers.

In one embodiment, the different layers have the following range of thicknesses: anode 110, 500-5000 Å, in one embodiment 1000-2000 Å; hole injection layer 120, 50-2000 Å, in one embodiment 200-1000 Å; hole transport layer 130, 50-2000 Å, in one embodiment 200-1000 Å; electroactive layer 140, 10-2000 Å, in one embodiment 100-1000 Å; layer 150, 50-2000 Å, in one embodiment 100-1000 Å; cathode 160, 200-10000 Å, in one embodiment 300-5000 Å. The location of the electron-hole recombination zone in the device, and thus the emission spectrum of the device, can be affected by the relative thickness of each layer. The desired ratio of layer thicknesses will depend on the exact nature of the materials used.

Depending upon the application of the device 100, the electroactive layer 140 can be a light-emitting layer that is activated by an applied voltage (such as in a light-emitting diode or light-emitting electrochemical cell), or a layer of material that responds to radiant energy and generates a signal with or without an applied bias voltage (such as in a photodetector). Examples of photodetectors include photoconductive cells, photoresistors, photoswitches, phototransistors, and phototubes, and photovoltaic cells, as these terms are described in Markus, John, Electronics and Nucleonics Dictionary, 470 and 476 (McGraw-Hill, Inc. 1966).

In some embodiments, the new deuterated compounds are useful as hole transport materials in layer 130. In some embodiments, at least one additional layer includes a deuterated material. In some embodiments, the additional layer is the hole injection layer 120. In some embodiments, the additional layer is the electroactive layer 140. In some embodiments, the additional layer is the electron transport layer 150.

In some embodiments, the new deuterated compounds are useful as host materials for electroactive materials in electroactive layer 140. In some embodiments, the emissive material is also deuterated. In some embodiments, at least one additional layer includes a deuterated material. In some embodiments, the additional layer is the hole injection layer 120. In some embodiments, the additional layer is the hole transport layer 130. In some embodiments, the additional layer is the electron transport layer 150.

In some embodiments, the new deuterated compounds are useful as electroactive materials in electroactive layer 140. In some embodiments, a host is also present in the electroactive layer. In some embodiments, the host material is also deuterated. In some embodiments, at least one additional layer includes a deuterated material. In some embodiments, the additional layer is the hole injection layer 120. In some embodiments, the additional layer is the hole transport layer 130. In some embodiments, the additional layer is the electron transport layer 150

In some embodiments, the new deuterated compounds are useful as electron transport materials in layer 150. In some embodiments, at least one additional layer includes a deuterated material. In some embodiments, the additional layer is the hole injection layer 120. In some embodiments, the additional layer is the hole transport layer 130. In some embodiments, the additional layer is the electroactive layer 140.

In some embodiments, an electronic device has deuterated materials in any combination of layers selected from the group consisting of the hole injection layer, the hole transport layer, the electroactive layer, and the electron transport layer.

In some embodiments, the devices have additional layers to aid in processing or to improve functionality. Any or all of these layers can include deuterated materials. In some embodiments, all the organic device layers comprise deuterated materials. In some embodiments, all the organic device layers consist essentially of deuterated materials.

### a. Electroactive layer

The new deuterated compounds described herein are useful as electroluminescent materials in layer 140. The compounds can be used alone, or in combination with a host material.

In some embodiments, the electroactive layer consists essentially of a host material and the new deuterated compound described herein.

In some embodiments, the host is a bis-condensed cyclic aromatic compound.

In some embodiments, the host is an anthracene derivative compound. In some embodiments the compound has the formula:

An - L- An

where:
An is an anthracene moiety;
L is a divalent connecting group.

In some embodiments of this formula, L is a single bond, -O-, -S-,-N(R)-, or an aromatic group. In some embodiments, An is a mono- or diphenylanthryl moiety.

In some embodiments, the host has the formula:

A-An-A

where:
An is an anthracene moiety;
A is the same or different at each occurrence and is an aromatic group.

In some embodiments, the A groups are attached at the 9- and 10-positions of the anthracene moiety. In some embodiments, A is selected from the group consisting naphthyl, naphthylphenylene, and naphthylnaphthylene. In some embodiments the compound is symmetrical and in some embodiments the compound is non-symmetrical.

In some embodiments, the host has the formula: 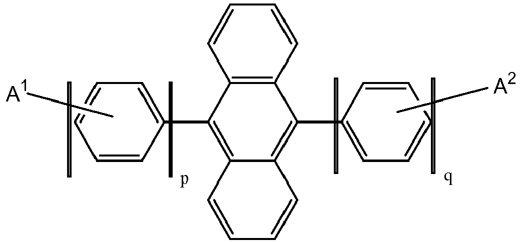 where:
A¹ and A² are the same or different at each occurrence and are selected from the group consisting of H, an aromatic group, an alkyl group and an alkenyl group, or A may represent one or more fused aromatic rings;
p and q are the same or different and are an integer from 1-3.

In some embodiments, the anthracene derivative is non-symmetrical. In some embodiments, p =2 and q = 1.

In some embodiments, at least one of A¹ and A² is a naphthyl group. In some embodiments, additional substituents are present.

In some embodiments, the host is selected from the group consisting of 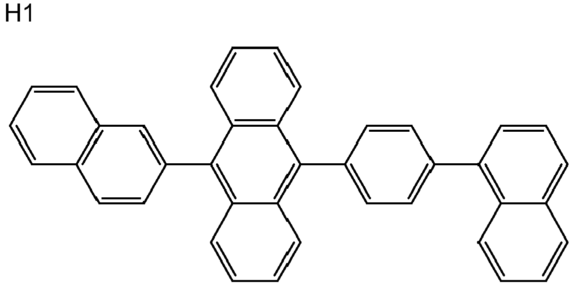 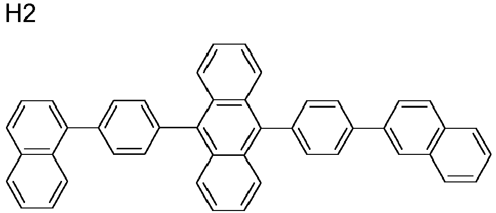 and combinations thereof.

The new deuterated compounds described herein, in addition to being useful as electroactive materials in the electroactive layer, can also act as charge carrying hosts for other electroactive materials in the electroactive layer 140. In some embodiments, the electroactive layer consists essentially of the new deuterated material and one or more electroactive materials.

### b. Other Device Layers

The other layers in the device can be made of any materials that are known to be useful in such layers.

The anode 110, is an electrode that is particularly efficient for injecting positive charge carriers. It can be made of, for example, materials containing a metal, mixed metal, alloy, metal oxide or mixed-metal oxide, or it can be a conducting polymer, or mixtures thereof. Suitable metals include the Group 11 metals, the metals in Groups 4-6, and the Group 8-10 transition metals. If the anode is to be light-transmitting, mixed-metal oxides of Groups 12, 13 and 14 metals, such as indium-tin-oxide, are generally used. The anode 110 can also comprise an organic material such as polyaniline as described in "Flexible light-emitting diodes made from soluble conducting polymer," Nature vol. 357, pp 477-479 (11 June 1992). At least one of the anode and cathode is desirably at least partially transparent to allow the generated light to be observed.

The hole injection layer 120 comprises hole injection material and may have one or more functions in an organic electronic device, including but not limited to, planarization of the underlying layer, charge transport and/or charge injection properties, scavenging of impurities such as oxygen or metal ions, and other aspects to facilitate or to improve the performance of the organic electronic device. Hole injection materials may be polymers, oligomers, or small molecules. They may be vapor deposited or deposited from liquids which may be in the form of solutions, dispersions, suspensions, emulsions, colloidal mixtures, or other compositions.

The hole injection layer can be formed with polymeric materials, such as polyaniline (PANI) or polyethylenedioxythiophene (PEDOT), which are often doped with protonic acids. The protonic acids can be, for example, poly(styrenesulfonic acid), poly(2-acrylamido-2-methyl-1-propanesulfonic acid), and the like.

The hole injection layer can comprise charge transfer compounds, and the like, such as copper phthalocyanine and the tetrathiafulvalene-tetracyanoquinodimethane system (TTF-TCNQ).

In some embodiments, the hole injection layer comprises at least one electrically conductive polymer and at least one fluorinated acid polymer. Such materials have been described in, for example, published U.S. patent applications 2004-0102577, 2004-0127637, and 2005/205860

In some embodiments, the new deuterated compounds described herein have utility as hole transport materials. Examples of other hole transport materials for layer 130 have been summarized for example, in Kirk-Othmer Encyclopedia of Chemical Technology, Fourth Edition, Vol. 18, p. 837-860, 1996, by Y. Wang. Both hole transporting molecules and polymers can be used. Commonly used hole transporting molecules are: N,N'-diphenyl-N,N'-bis(3-methylphenyl)-[1,1'-biphenyl]-4,4'-diamine (TPD), 1,1-bis[(di-4-tolylamino) phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis-(3-methylphenyl)-N,N,N',N'-2,5-phenylenediamine (PDA), a-phenyl-4-N,N-diphenylaminostyrene (TPS), p-(diethylamino)benzaldehyde diphenylhydrazone (DEH), triphenylamine (TPA), bis[4-(N,N-diethylamino)-2-methylphenyl](4-methylphenyl)methane (MPMP), 1-phenyl-3-[p-(diethylamino)styryl]-5-[p-(diethylamino)phenyl] pyrazoline (PPR or DEASP), 1,2-trans-bis(9H-carbazol-9-yl)cyclobutane (DCZB), N,N,N',N'-tetrakis(4-methylphenyl)-(1,1'-biphenyl)-4,4'-diamine (TTB), N,N'-bis(naphthalen-1-yl)-N,N'-bis-(phenyl)benzidine (α-NPB), and porphyrinic compounds, such as copper phthalocyanine. Commonly used hole transporting polymers are polyvinylcarbazole, (phenylmethyl)-polysilane, and polyaniline. It is also possible to obtain hole transporting polymers by doping hole transporting molecules such as those mentioned above into polymers such as polystyrene and polycarbonate. In some cases, triarylamine polymers are used, especially triarylamine-fluorene copolymers. In some cases, the polymers and copolymers are crosslinkable. Examples of crosslinkable hole transport polymers can be found in, for example, published US patent application 2005-0184287 and published PCT application WO 2005/052027. In some embodiments, the hole transport layer is doped with a p-dopant, such as tetrafluorotetracyanoquinodimethane and perylene-3,4,9,10-tetracarboxylic-3,4,9,10-dianhydride.

In some embodiments, the new deuterated compounds described herein have utility as electron transport materials. Examples of other electron transport materials which can be used in layer 150 include metal chelated oxinoid compounds, such as tris(8-hydroxyquinolato)aluminum (Alq₃); bis(2-methyl-8-quinolinolato)(para-phenyl-phenolato)aluminum(III) (BAIQ); and azole compounds such as 2-(4-biphenylyl)-5-(4-t-butylphenyl)-1,3,4-oxadiazole (PBD) and 3-(4-biphenylyl)-4-phenyl-5-(4-t-butylphenyl)-1,2,4-triazole (TAZ), and 1,3,5-tri(phenyl-2-benzimidazole)benzene (TPBI); quinoxaline derivatives such as 2,3-bis(4-fluorophenyl)quinoxaline; phenanthroline derivatives such as 9,10-diphenylphenanthroline (DPA) and 2,9-dimethyl-4,7-diphenyl-1,10-phenanthroline (DDPA); and mixtures thereof. The electron-transport layer may also be doped with n-dopants. N-dopant materials are well known. The n-dopants include, but are not limited to, Group 1 and 2 metals; Group 1 and 2 metal salts, such as LiF, CsF, and Cs₂CO₃; Group 1 and 2 metal organic compounds, such as Li quinolate; and molecular n-dopants, such as leuco dyes, metal complexes, such as W₂(hpp)₄ where hpp=1,3,4,6,7,8-hexahydro-2H-pyrimido-[1,2-a]-pyrimidine and cobaltocene, tetrathianaphthacene, bis(ethylenedithio)tetrathiafulvalene, heterocyclic radicals or diradicals, and the dimers, oligomers, polymers, dispiro compounds and polycycles of heterocyclic radical or diradicals. Layer 150 can function both to facilitate electron transport, and also serve as a hole injection layer or confinement layer to prevent quenching of the exciton at layer interfaces. Preferably, this layer promotes electron mobility and reduces exciton quenching.

The cathode 160, is an electrode that is particularly efficient for injecting electrons or negative charge carriers. The cathode can be any metal or nonmetal having a lower work function than the anode. Materials for the cathode can be selected from alkali metals of Group 1 (e.g., Li, Cs), the Group 2 (alkaline earth) metals, the Group 12 metals, including the rare earth elements and lanthanides, and the actinides. Materials such as aluminum, indium, calcium, barium, samarium and magnesium, as well as combinations, can be used. Li- or Cs-containing organometallic compounds, LiF, CsF, and Li₂O can also be deposited between the organic layer and the cathode layer to lower the operating voltage.

It is known to have other layers in organic electronic devices. For example, there can be a layer (not shown) between the anode 110 and hole injection layer 120 to control the amount of positive charge injected and/or to provide band-gap matching of the layers, or to function as a protective layer. Layers that are known in the art can be used, such as copper phthalocyanine, silicon oxy-nitride, fluorocarbons, silanes, or an ultra-thin layer of a metal, such as Pt. Alternatively, some or all of anode layer 110, active layers 120, 130, 140, and 150, or cathode layer 160, can be surface-treated to increase charge carrier transport efficiency. The choice of materials for each of the component layers is preferably determined by balancing the positive and negative charges in the emitter layer to provide a device with high electroluminescence efficiency.

It is understood that each functional layer can be made up of more than one layer.

The device can be prepared by a variety of techniques, including sequential vapor deposition of the individual layers on a suitable substrate. Substrates such as glass, plastics, and metals can be used. Conventional vapor deposition techniques can be used, such as thermal evaporation, chemical vapor deposition, and the like. Alternatively, the organic layers can be applied from solutions or dispersions in suitable solvents, using conventional coating or printing techniques, including but not limited to spin-coating, dip-coating, roll-to-roll techniques, ink-jet printing, screenprinting, gravure printing and the like.

To achieve a high efficiency LED, the HOMO (highest occupied molecular orbital) of the hole transport material desirably aligns with the work function of the anode, and the LUMO (lowest un-occupied molecular orbital) of the electron transport material desirably aligns with the work function of the cathode. Chemical compatibility and sublimation temperature of the materials are also important considerations in selecting the electron and hole transport materials.

It is understood that the efficiency of devices made with the chrysene compounds described herein, can be further improved by optimizing the other layers in the device. For example, more efficient cathodes such as Ca, Ba or LiF can be used. Shaped substrates and novel hole transport materials that result in a reduction in operating voltage or increase quantum efficiency are also applicable. Additional layers can also be added to tailor the energy levels of the various layers and facilitate electroluminescence.

The compounds of the invention often are photoluminescent and can be useful in applications other than OLEDs, such as oxygen sensitive indicators and as fluorescent indicators in bioassays.

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification and figures are to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination. Further, reference to values stated in ranges include each and every value within that range

## Claims

1. A compound having Formula VIII-a or VIII-b. 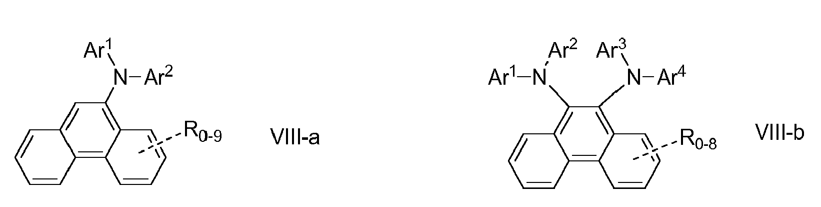 wherein:
R is the same or different at each occurrence and is selected from the group consisting of D, alkyl, alkoxy and aryl, where adjacent R groups may be joined together to form a 5- or 6-membered aliphatic ring;
Ar¹ through Ar⁴ are the same or different and are selected from the group consisting of aryl groups;
wherein the compound has at least one D.

2. The compound of Claim 1, having at least 20% deuteration.

3. The compound of Claim 1, wherein deuteration is on a substituent group on an aryl ring.

4. The compound of Claim 1, wherein deuteration is on any one or more of Ar¹ through Ar⁴.

5. The compound of Claim 1, wherein deuteration is on the phenanthrene moiety.

6. The compound of Claim 1, wherein at least one R is a hydrocarbon alkyl, which is optionally deuterated.

7. The compound of Claim 1, wherein R is selected from a branched hydrocarbon alkyl, a cyclic hydrocarbon alkyl, and deuterated analogs thereof.

8. The compound of Claim 1, wherein at least one of A¹ through A⁴ has formula (a): 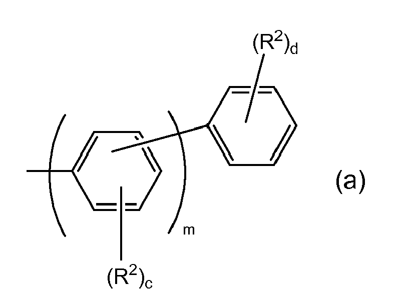 where:
R² is the same or different at each occurrence and is selected from the group consisting of D, alkyl, alkoxy, aryl, silyl, and siloxane, or adjacent R² groups can be joined to form an aromatic ring;
c is the same or different at each occurrence and is an integer from 0-4;
d is the same or different at each occurrence and is an integer from 0-5; and
m is the same or different at each occurrence and is an integer from 0 to 6.

9. The compound of Claim 1, wherein at least one of A¹ through A⁴ has formula (b): 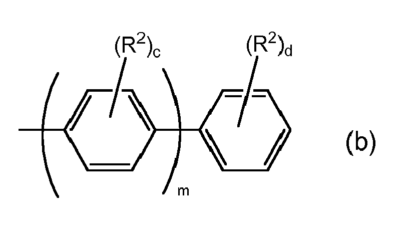 where:
R² is the same or different at each occurrence and is selected from the group consisting of D, alkyl, alkoxy, and aryl, or adjacent R² groups can be joined to form an aromatic ring;
c is the same or different at each occurrence and is an integer from 0-4;
d is the same or different at each occurrence and is an integer from 0-5; and
m is the same or different at each occurrence and is an integer from 0 to 6.

10. The compound of Claim 1, wherein A¹ through A⁴ are selected from the group consisting of phenyl, biphenyl, terphenyl, naphthyl, phenylnapthyl, naphthylphenyl, binaphthyl, and deuterated analogs thereof.

11. The compound of Claim 1, wherein A¹ through A⁴ are perdeuterated, optionally except for one alkyl group on a terminal aryl.

12. The compound of Claim 1, wherein the compound is not symmetrical with respect to the Ar groups and Ar¹ is not the same as either Ar³ or Ar⁴.

13. An organic electronic device having one or more layers comprising a compound of any preceding claim.

14. A device of Claim 13, wherein the device is selected from:
one that converts electrical energy into radiation, one that detects signals through an electronic process, one that converts radiation into electrical energy, and one that includes one or more electronic components that include one or more organic semi-conductor layers.

15. A device (100) of Claim 13 or 14 that comprises a first electrical contact layer (anode layer 110); a second electrical contact layer (cathode layer 160), and an electroactive layer (140) between them, whereby adjacent to the anode is a hole injection layer (120) adjacent to the hole injection layer in a hole transport layer (130) comprising a hole transport material and adjacent to the cathode may be an electron transport layer (150) comprising an electron transport material, optionally with one or more additional hole transport or hole injection layers next to the anode and/or one or more additional electron transport or electron injection layers next to the cathode.
